# EUROPEAN PATENT APPLICATION

(11) **EP 2 508 229 A1**
(43) Date of publication of application: **10.10.2012**
(21) Application number: 11161534.0
(22) Date of filing: 07.04.2011
(51) Int. Cl.: A61N 5/06

(54) **Method and device for biostimulating irradiation**

(71) Applicant: Koninklijke Philips Electronics N.V., 5621 BA Eindhoven (NL)
(72) Inventor: Oversluizen, Gerrit, 5600 AE, Eindhoven (NL); Dekker, Tim, 5600 AE, Eindhoven (NL); Van Abeelen, Frank, A., 5600 AE, Eindhoven (NL); Van Pieterson, Liesbeth, 5600 AE, Eindhoven (NL); Lemmens, Paul, M., C., 5600 AE, Eindhoven (NL)
(74) Representative: Van Eeuwijk, Alexander Henricus Waltherus

(57) **Abstract**

A method for biostimulation of a body portion of a subject to be treated is herewith provided, comprising irradiating the skin of the body portion to be treated with a first dose of light so as to heat a surface layer of the skin with a rate of temperature increase of at least about 0,04 degrees Celsius per second and irradiating the body portion with a second dose of light as a function of a property of the body portion indicative of vasodilatation. A device for use in the method is also provided. The device comprises a light source. A portion of the device is a patch which is formed for conforming to at least part of the body portion. The device is configured to irradiate the body portion and thereby provide a temperature increase of a surface layer of the skin at a rate of at least 0,04 degrees Celsius per second. The device further comprises a sensor for measuring at least one parameter of the body portion indicative of a vasodilatation.

## Description

### TECHNICAL FIELD

The present disclosure relates to the field of biostimulation, e.g. biostimulating irradiation. Particular interest is to devices and methods for purposes of dermatological phototherapy, pain relief, wound healing and/or dermal health. The device and methods are suitable both in professional and domestic use, and may be used for curative, cosmetic and/or wellness purposes.

### BACKGROUND

It is known that vasodilatation, an increase in the size of blood vessels leading to increased blood perfusion, is part of the sophisticated thermal regulation system of the human body. Aside from regulating temperature, vasodilatation is considered beneficial for processes of pain relief, wound healing and/or dermal health. It is believed that improved perfusion resulting from vasodilatation provides and/or improves a fair skin and a healthy complexion.

Vasodilatation may be caused by heat. However, optical stimulation may also cause vasodilatation. E.g., US 2009/0204185 discloses that light of particular wavelengths stimulates perfusion of the illuminated tissue by light-induced blood vessel and lymph vessel vasodilatation, and that this may reduce inflammation of the illuminated tissue. Further, the immune system is stimulated to protect the body from inflammation by light-induced NO synthesis.

There is a desire to improve the efficiency of biostimulation further and in particular to improve the process of exciting and/or maintaining vasodilatation. A further desire is to increase energy efficiency of a biostimulation device, in particular a patch.

### SUMMARY

A method for biostimulation of a body portion of a subject to be treated is hereby provided. The method comprises irradiating the skin of the body portion to be treated with electromagnetic radiation, in particular UV/blue or infrared, light so as to heat a surface layer of the skin with a rate of temperature increase of at least about 0,04 degrees Celsius per second, which may be written as dT/dt > ca. 0,04 °C/s, wherein "dT/dt" denotes the derivative of temperature T to time t.

Local heating of the skin evokes cutaneous vasodilatation, where both the rate of temperature change, in particular temperature increase, and the absolute skin temperature are important The stronger the stimulus, the more effective it proves for producing the physiological effect. A fast vasodilatation response is mediated by exciting nociceptors acting in an axon reflex. and it has presently been found that optical techniques may be particularly efficient to excite the nociceptors.

It has been found that with such irradiation of the skin for a short time, which may be on the order of seconds, e.g. some tens of seconds but generally less than about a few minutes, preferably less than about one minute, suffices to trigger the physiologic thermal response. The duration and intensity of the irradiation relate to the amplitude of the response (amount of vasodilatation etc.) as will be detailed below. Moreover, it has been found that once the thermal response of the skin is triggered, the response is maintained for prolonged duration, generally several minutes, without further stimulation of the heat receptors. Thus, a single dose of the radiation, comprising a relatively short exposure, may suffice to provide a relatively long period of vasodilatation. At least a portion of the radiation may be administered as a pulsed dose.

Therefore, vasodilatation is excited and maintained by administration of a first irradiation pulse to excite the vasodilatation response to a predetermined first level and by administration of a second irradiation pulse, comprising a second dose of radiation, when the response has decreased to a second predetermined level.

Advantageously, the second irradiation pulse is configured to excite the vasodilatation (again) to the predetermined first level. The level first and second predetermined level may be determined compared to a base value, e.g. with respect to the subject being comfortably at rest at room temperature, e.g. as an increase of the blood flow volume by a predetermined factor e.g. a factor of 1,5, 2, 2,5 or 3, and may be selected to be close to each other.

Heating the skin may be provided with other means than light, e.g. see W.Magerl and R-D.Treede, J.Physiol. (1996)497.3, pp837-848, but providing the heat by treatment with light has numerous benefits, effective independently or in combination. E.g., selection of a particular wavelength range allows selecting a suitable penetration depth of the radiation into skin. Also, a light source may generally be switched on and off at will. Thus, the stimulus may be provided accurately on demand with little to no rise time and/or decay time. Most light sources also allow accurate control over their emitted power. Light sources may be small in volume yet can provide their effect (irradiation) over relatively large surface areas.

Therefore, the method provided herewith has improved efficiency and reliability in exciting the vasodilatation response in a body portion in normal state, i.e. with normal perfusion, e.g. with respect to factors such as exciting the vasodilatation response, onset and increase of the response, extent and duration of the response etc.

Vasodilatation may be determined with different techniques, e.g. using plethysmography, laser Doppler flowmetry, skin thermometry, determination of the rate of change of skin temperature relative to an applied temperature or temperature change, have all been proven efficient methods for reliably determining vasodilatation. Another method is measuring skin color and in particular erythema, i.e. red-coloring of the skin, which however tends to lag the onset and decay of the vasodilatation as determined in other ways and which in some subjects may be difficult to detect. Further, a chemical sensor may be provided, e.g. for detecting nitric oxide (NO). The skin surface temperature of an average healthy human adult at room temperature generally is about 30-35 °C, for most people about 31-33 °C. Generally, temperatures above 38 °C are considered warm, above 42 °C is considered hot and pain and/or tissue damage may occur at temperatures of about 45 °C and higher. For biostimulation, the skin temperature should therefore preferably be (maintained) at a temperature below about 42 °C. The present method facilitates reliably achieving vasodilatation without overheating the irradiated skin. The present method may therefore be used for domestic use e.g. for cosmetic and wellness purposes. As an example, it is believed that increased blood perfusion of skin assists in reducing aging effects and enhancing its rejuvenation.

The method may be combined with further treatments having a medical, cosmetic and/or wellness nature. Such further treatment may comprise phototherapy. The radiation used for the method may in itself also have biostimulating effects.

Further, the faster the change is, the smaller is the temperature difference required to experience a thermal sensation and to excite vasodilatation. E.g., prolonged heating of a skin portion with a temperature increase rate of about 0,01 °C/s, has proven all but ineffective in triggering vasodilatation, at least up to skin temperatures well over 39 °C. At a thermal increase rate of 0,02 °C/s the vasodilatation response may start, but only slowly and with little reliability. The presently claimed values reliably excite vasodilatation in substantially all subjects.

In an advantageous embodiment, the temperature increase rate may be at least about 0,07 °C/s, preferably at least about 0,1 °C/s, more preferably at least about 0,3 °C/s.

Faster heating rates may also be energy efficient for a treatment as a whole, since the accelerated and increased vasodilatation response may require in total less energy for heating to a lower final temperature of the skin layer to achieve a satisfactory reaction, so that the product of optical power times the treatment duration that need be deposited in the skin may be reduced.

In accordance with the above, a device for biostimulation of a body portion of a subject to be treated is provided herewith. The device comprises a light source and it is configured to irradiate the skin of the body portion. At least a portion of the device is formed for conforming to at least part of the body portion, e.g. by comprising a flexible, pliable or generally deformable portion such as a patch or bandage. The device being formed for conforming to at least part of the body portion to be treated improves user comfort and facilitates prolonged treatment. Such device, in particular in the form of a patch or bandage, may be worn inconspicuously under clothing. Such device allows improved and predictable irradiation of the body portion since shifted irradiation portions and/or shadows caused by relative movement of the device and the body portion are prevented. The device is configured to irradiate the body portion and thereby provide a temperature increase of a surface layer of the skin at a rate of at least 0,04 degrees Celsius per second. The device further comprises a sensor for measuring at least one parameter of the body portion indicative of a vasodilatation.

The device may comprise any suitable sensor for measuring a parameter of the body portion indicative of vasodilatation, in particular a sensor for sensing at least one skin property. A thermometer to detect and/or monitor temperature of the skin of the body portion is preferred, in particular of the skin surface. Such thermometer may be an intradermal thermometer, but a non-invasive detector such as a contact thermometer or an optical thermometer is preferred.

The device may comprise a controller configured to operate the device, e.g. the light source.

The controller may be configured to operate the device, as a function of a signal of the sensor, which may relate to a parameter determined by the sensor, e.g. the redness, skin temperature and/or electrical conductivity of the skin. It is preferred that the controller is connected and/or connectable to a time keeping device, e.g. a clock and/or a timer, which may be integrated in the device or even in the controller. Thus, the rate of temperature change of the skin may be determined. Further, this facilitates feedback on the operation of the device and/or on the efficiency of the treatment. E.g., the power of the light source may be controlled, e.g. with a feedback-loop, to provide a predetermined rate of skin temperature change, to stop operation when reaching a particular skin temperature and/or amount of vasodilatation and/or a particular NO production.

Advantageously, the patch is configured to provide a temperature increase at a rate of at least about 0,07 °C/s, preferably at least about 0,1 °C/s, more preferably at least about 0,3 °C/s.

To increase control over the heating and reduce chances of accidental overheating it is preferred that the rate of temperature increase be kept below a value of 2 °C/s_and that the heating power of the device be correspondingly configured to provide at most such rate of temperature increase, e.g. providing a fixed or user selectable maximum heating power.

An advantageous embodiment of the method comprises irradiating the body portion over a relatively large surface area of well over 15 cm², preferably well over 25 cm², more preferably over 50 cm² and most preferably over 100 cm².

And an efficient device may similarly be configured for irradiating the body portion over a surface area of at least about 15 cm², preferably at least about 25 cm², more preferably at least about 50 cm² and most preferably at least about 100 cm².

The larger the irradiated area is, the stronger is the stimulus. It is found that an increase in irradiated surface area produces a larger increase in the effectiveness of exciting vasodilatation, so that a lower temperature increase and/or rate of change triggers the response. Hence, an increased energy demand for increasing the irradiated surface area may be outweighed by a reduced treatment time or temperature increase, so that the energy demand of a treatment as a whole decreases.

Irradiation of such surface area may be achieved in various ways, e.g. by a light source having a large emitting surface area, e.g. a large-area OLED, a compound light source comprising plurality of individual light sources having relatively smaller surfaces area, e.g. an LED-array, and/or an optical element to distribute the light over the surface area to be irradiated. Advantageously, such optical element comprises a light guide, which may comprise one or more flexible portions and/or reflective portions. Incoherent light sources are preferred over lasers or superluminal light sources, since these require additional control, increasing complexity and cost of the device. Also, relatively narrowband radiation poses an increased risk of overheating skin. Laser radiation may also present a danger to users eyes.

An advantageous embodiment of the method comprises irradiating the body portion with light having a wavelength in a range of about 300-600 nm being blue to ultraviolet ("UV") and/or 900-10000 nm, being near infrared ("IR"). An efficient device may be configured to emit light in such wavelength range. Advantageously, the wavelengths to be used are, on the blue/UV side, in a range of about 350-550 nm, preferably about 400-500 nm, e.g. about 450-470 nm, and/or on the IR side, in a range of about 1200-5000 nm, preferably about 1300-2000 nm, e.g. about 1500-1800 nm.

It has been found that light at such wavelength ranges has relatively little penetration depth into the skin, less than about 1 mm, and is absorbed predominantly in the epidermis where most receptors (thermoreceptors and nociceptors) are located. Thus, superficial heating of the skin is sufficient and efficiency of the therapy is improved. Also, responsiveness to the method may be improved, since time lag due to slow spreading and/or heat capacity of subdermal tissue is prevented. Further, thermal load of subdermal tissue due to the irradiation may be reduced or even prevented.

The method may comprise irradiating the body portion with an irradiation power density in a range of about 20-200 mW/cm², preferably more than about 50 mW/cm² and more preferably more than about 100 mW/cm², e.g. about 150 mW/cm².

The device may be configured to provide an irradiation power density in such range.

10 mW/cm² irradiation power density is generally the lower level of what the skin receptors can detect as a heat input. 40 mW/cm² time averaged intensity is generally the power density that a healthy skin can absorb and lose via re-emission and/or convection, but which may lead to some heating with. Correspondingly, the heating rate may be low and/or unpredictable. A power density of about 50 mW/cm² therefore is considered a lower bound for achieving a reliable stimulus. It has been found that, the higher the power density, the faster the vasodilatation effect is excited and/or the stronger the effect will be. Irradiating with a higher power may therefore require less energy in total to excite the vasodilatation response than using a lower power for a longer period.

The power density may be achieved with a time-average power density of a pulse train comprising a plurality of short pulses individually providing a higher power density, wherein the average is considered over a suitable time, e.g. over a period of about 5-30 seconds, typically about 15 seconds and in particular as a moving average taken over about 5-10 seconds. However, prolonged exposure to irradiation at power densities of above 200 mW/cm², e.g. for tens of seconds to several minutes may cause pain and in extreme cases (very high pulse powers and/or very long exposure) tissue damage, even if not reaching the maximum temperature of about 43-45 °C which are to be avoided in the presently considered realm ofbiostimulation.

The method may comprise irradiating the body portion with a plurality of pulses of the biostimulating light, e.g. for increasing or maintaining the level of vasodilatation compared to a particular level, in particular a base level determined with (the body portion of) the subject to be treated at rest in the ambient temperature of the environment where the method is to be performed, typically room temperature.

The method may comprise administrating a plurality of doses of the biostimulating light as a function of one or more properties of (the skin of) the body portion, in particular in accordance with a measured and/or a predicted variation in one or more parameters of the body portion indicative of vasodilatation.

Summarizing, it has been found that with the present method and device vasodilatation may be excited within tens of seconds using a sufficiently strong stimulus. Typically, vasodilatation is clearly detectable, at least via laser Doppler flowmetry, within a few seconds after excitation. The maximum level of the effect depends on the strength and duration of the stimulus, but generally, the vasodilatation once excited reaches a plateau and decays only very slowly over several minutes after removing the stimulus.

The disclosed method of applying a series of pulses to excite and maintain vasodilatation within a predetermined range or "window" is significantly more energy-efficient than continuously irradiating the body portion. A device operating according to the method may therefore have improved efficacy. E.g., an exemplary embodiment of a battery-powered patch may have extended operating life time on a single charge.

In a particular embodiment, the body portion may be irradiated with the biostimulating light in a pulse train with alternating periods of high intensity and little to zero irradiation (no light) and the skin temperature may be measured optically in between the pulses at the periods of little or zero irradiation. Thus, temperature may be accurately measured without "contamination" of the measurement by the irradiating light and/or physical contact between the thermometer and the skin. Such "interleaved" irradiation and measuring sequences may be controlled by a controller.

### BRIEF DESCRIPTION OF THE DRAWINGS

The above-described aspects will hereafter be more explained with further details and benefits with reference to the drawings showing an embodiment of the invention by way of example.
Fig. 1 is a schematic representation of irradiation of the skin of a human body portion;
Figs. 2 and 3 are schematic side views of different devices.

### DETAILED DESCRIPTION OF EMBODIMENTS

It is noted that in the drawings, like features may be identified with like reference signs. It is further noted that the drawings are schematic, not necessarily to scale and that details that are not required for understanding the present invention may have been omitted. The terms "upward", "downward", "below", "above", and the like relate to the embodiments as oriented in the drawings, unless otherwise specified. Further, elements that are at least substantially identical or that perform an at least substantially identical function are denoted by the same numeral.

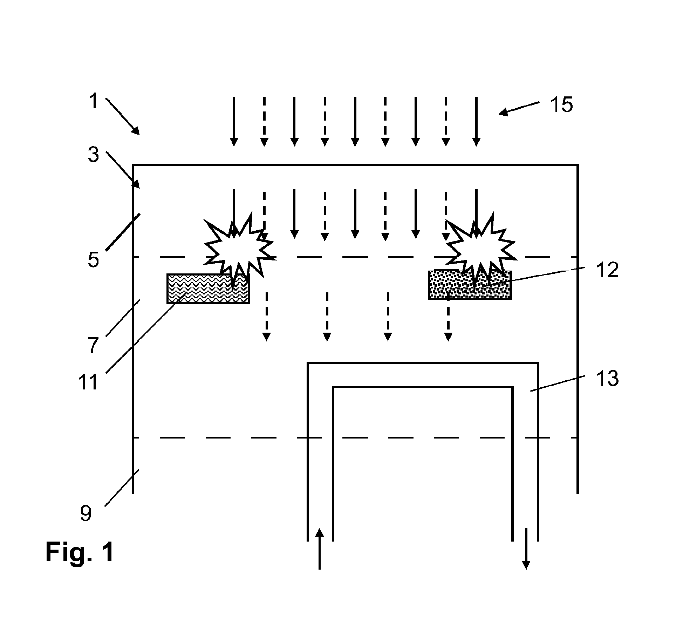

Fig. 1 illustrates irradiation of a human body portion 1, having a skin portion 3 which comprises an epidermis layer 5 of ca 0.1-1.5 mm thickness (dependent on the person and the location of the body), and a ca 1-4 mm thick dermis layer 7 and which covers hypodermic tissue 9. To keep thermal equilibrium, the human body has a sophisticated thermal regulation system. On a local level, this is mainly controlled by thermoreceptors 11 and nociceptors 12 that are located in the outer layers of the skin at 0.2 mm - 0.5 mm below the surface. The dermis 7 further comprises capillary blood vessels 13 connected to larger blood vessels deeper in the body portion (not shown). Fig. 1 further shows irradiation light 15 having a wavelength in a range of 300-600 nm and/or 900-10000 nm which is transmitted into the epidermis 5 and is absorbed there (indicated with a small star). Light having a wavelength in a wavelength range between 600-900 nm is generally transmitted through the epidermis 5 and into the dermis 7 (or deeper, not shown) and it will be absorbed there (indicated with dashed arrows).

The light 15 excites the thermoreceptors 11 and the nociceptors 12, either directly or indirectly by heating the epidermis 5. Sufficient excitation of the sensors 11, 12 in the skin triggers the vasodilatation, increased blood flow, and possibly sweating. Since direct excitation of the nociceptors is hard to measure, in particular non-invasively or minimum-invasively (e.g. with a single intradermal or transdermal probe just like an injection needle), heating of the skin surface layer, is considered an accurate indicator, in particular the rate of increase of the skin temperature.

Suitable irradiation power densities to trigger the response comprise administering a dose with an intensity in the range of approximately 50-200 mW/cm², for a duration of approximately 5-30 seconds for a wavelength in the range of 900-10000 nm, e.g. 100 mW/cm² for about 10 seconds at 1550 nm, e.g. using a number of telecommunications LEDs. For wavelengths in the range 400-600 nm generally lower doses suffice; a wavelength of about 450 nm, with a spectral bandwidth of ca. 50 nm around a centre wavelength of ca. 450 nm, has been found to be particularly efficient in exciting vasodilatation. It also has a beneficial effect of NO production in most subjects. The irradiation may be administered via a series of short high-intensity pulses and at a high pulse repetition rate. The actual heating of the skin may depend on the absorption characteristics and/or reflection characteristics of the skin, which may depend from one person to the next. However, the rate of skin temperature increase has been found to be a reliable parameter for different skin types.

Once the vasodilatation response is active, the irradiation may be stopped, removing the stimulus, whereas the vasodilatation response lingers for tens of seconds to minutes. In some cases the response lags somewhat and continues to increase even after removal of the stimulus.

Generally, vasodilatation has a starting phase wherein the blood vessels start to dilate, a plateau phase wherein the blood vessels reach and maintain a maximum dilated size, and a decay phase wherein the skin returns to the starting condition. These phases may be monitored by the above-referenced various measuring techniques.

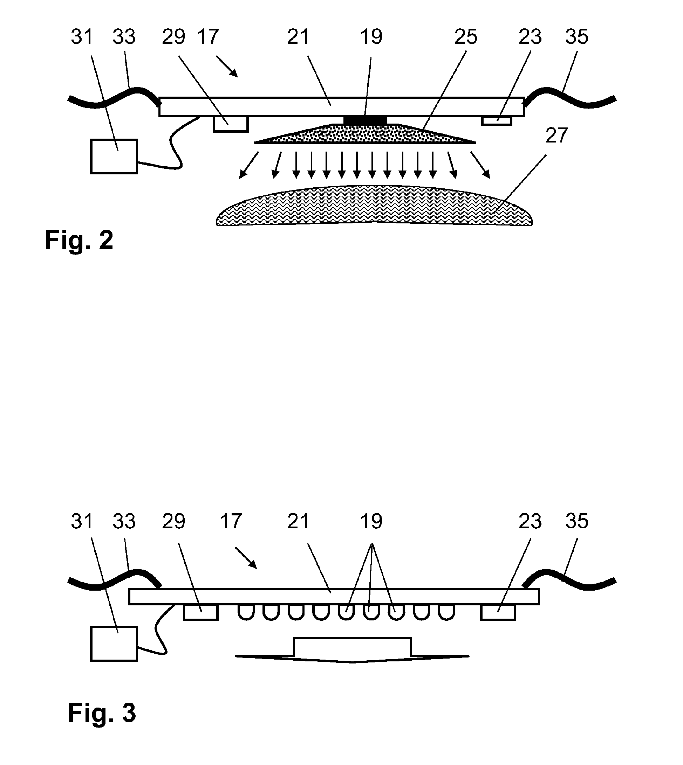

Fig. 2 shows a suitable device 17 for use in the above-described method. The device 17 comprises a light source 19, e.g. an LED, for providing light at a bio-stimulating phototherapeutic wavelength mounted to a carrier 21, and a sensor 23, e.g., an optical thermometer. Further, a light guide 25 is optically coupled to the light source 19. The device 17 is configured to irradiate a body portion 27 of a subject with light emitted by the light sources 19, transmitted and redistributed through the light guide 25 (see small arrows). The device 17 further comprises a controller 29 connected with the sensor 23 for controlling operation of the light source 19 as a function of a skin property determined by the sensor 23, e.g. the temperature of the irradiated skin of the body portion 27.

The device 21 may be powered from any suitable power source 31, for portability powering from an accumulator or battery (not shown) is preferred. Since the device 17 is configured for energy efficient excitation and maintenance of vasodilatation in the body portion 27, battery operation for prolonged periods is allowed.

A portion of the device 21 is formed as a human wearable patch, such as a device conforming to a human body portion, e.g. an arm, a leg, a back etc., preferably being deformable or even pliable. For this, the carrier 21 and/or the light guide 25 may be deformable, e.g. comprising a textile or silicone material. The patch may be maintained in position with any suitable means such as one or more adhesive portions, hook-and-loop-type fastener and/or straps 33, 35 closable around the body portion.

Fig. 3 shows an alternative patch 17, comprising a plurality of light sources 19, mounted to a flexible carrier 21. The combined light cones of the individual light sources 19 provide irradiation over a large surface area (indicated with the broad arrow). Such construction facilitates forming the patch around a body portion and irradiating a large surface area.

Light Emitting Diodes or LEDs are available for numerous suitable wavelengths, provide significant optical output power per watt input power and generate little heat. However, the heat that is generated by the light sources, also in the case of LEDs may be used to the advantage as an additional heat source, since the light sources or portions of the patch between adjacent light sources may serve as black body radiators, e.g. a temperature of about 50 °C corresponds to a peak wavelength of about 9000 nm and 70 °C corresponds to about 8500 nm. Such wavelengths are also chiefly absorbed in the epidermis.

The light sources 19 may be at least partially surrounded or embedded in a padding material to increase user comfort. The device may be provided with a transparent wrapping or cover, which may be washable. A disposable cover may increase hygiene.

The controller may comprise a user interface with selectable settings, e.g. heating rates and/or a maximum skin temperature. Also, the controller may be configured to take additional input, e.g. for determining parameters of a treatment, one or more user settings, timings, etc. Advantageously, the controller is programmable and/or programmed for controlling operation of the light source. Such program may be stored on or in a memory comprised in the device.

A light guide may comprise ridges, bumps, reflective and/or lens portions. A light guide may comprise one or more fiberoptic portions, e.g. one or more fiber bundles for redistributing the light.

Other variations to the disclosed embodiments can be understood and effected by those skilled in the art in practicing the claimed invention, from a study of the drawings, the disclosure, and the appended claims. Features from different embodiments may be suitably combined within the scope of the appended claims, unless explicitly mentioned otherwise. "Light emitting diode" or LED includes "organic light emitting diode" or OLED. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

Elements and aspects discussed for or in relation with a particular embodiment may be suitably combined with elements and aspects of other embodiments, unless explicitly stated otherwise.

## Claims

1. A method for biostimulation of a body portion of a subject to be treated, comprising
irradiating the skin of the body portion to be treated with a first dose of light
so as to heat a surface layer of the skin with a rate of temperature increase of at least about 0,04 degrees Celsius per second,
and irradiating the body portion with a second dose of light as a function of a property of the body portion indicative of vasodilatation.

2. The method of claim 1, comprising determining a property of the body portion indicative of vasodilatation.

3. The method of claim 1, wherein the rate of temperature increase is at least about 0,07 degrees Celsius per second, preferably at least about 0,1 °C/s, more preferably at least about 0,3 °C/s.

4. The method of claim 1, comprising irradiating the body portion over a surface area of at least about 15 cm², preferably at least about 25 cm², more preferably at least about 50 cm² and most preferably at least about 100 cm².

5. The method of claim 1, comprising irradiating the body portion with light having a wavelength in a range of about 300-600 nm and/or 900-10000 nm, preferably in a range of about 350-550 nm, more preferably in a range of about 400-500 nm, e.g. about 450-470 nm, and/or in a range of about 1200-5000 nm, preferably in a range of about 1300-2000 nm, e.g. in a range of about 1500-1800 nm.

6. The method of claim 1, comprising irradiating the body portion at a power density in a range of about 20-200 mW/cm².

7. The method of claim 1, comprising irradiating the body portion with a dose of the light as a function of a measured and/or a predicted variation in one or more parameters of the body portion indicative of vasodilatation.

8. A device for use in the method of claim 1, comprising a light source for biostimulation of a body portion of a subject to be treated,
wherein at least a portion of the device is a patch which is formed for conforming to at least part of the body portion and
wherein the device is configured to irradiate the body portion and thereby provide a temperature increase of a surface layer of the skin at a rate of at least 0,04 degrees Celsius per second,
wherein the device comprises a sensor for measuring at least one parameter of the body portion indicative of a vasodilatation.

9. The device according to claim 8, comprising a controller for controlling operation of the patch.

10. The device according to claim 9, wherein the controller is configured for controlling operation of the patch in response to at least one signal of the sensor.

11. The device according to claim 10, wherein the sensor is a thermometer and the controller is at least one of connected and/or connectable to a time keeping device.

12. The device according to claim 8, wherein the device is configured to provide a temperature increase of at least about 0,07 degrees Celsius per second, preferably at least about 0,1 °C/s, more preferably at least about 0,3 °C/s.

13. The device according to claim 8, configured for irradiating the body portion over a surface area of at least about 15 cm², preferably at least about 25 cm², more preferably at least about 50 cm² and most preferably at least about 100 cm².

14. The device according to claim 8, configured to emit light having a wavelength in a range of about 300-600 nm and/or 900-10000 nm, preferably in a range of about 350-550 nm, 400-500 nm, e.g. about 450-470 nm, and/or in a range of about 1200-5000 nm, preferably about 1300-2000 nm, e.g. about 1500-1800 nm.

15. The device according to claim 8, configured to emit light with a power density in a range of about 20-200 mW/cm2.
